# EUROPÄISCHE PATENTANMELDUNG

(11) **EP 3 502 274 A1**
(43) Veröffentlichungstag der Anmeldung: **26.06.2019**
(21) Anmeldenummer: 17210121.4
(22) Anmeldetag: 22.12.2017
(51) Int. Cl.: C12Q 1/6806, C12N 11/08, C12M 1/00, G01N 33/569

(54) **PROBENTRÄGER UND VERFAHREN ZU DESSEN HERSTELLUNG**

(71) Anmelder: Attomol GmbH, 03205 OT Lipten, Bronkow (DE)
(72) Erfinder: LEHMANN, Werner, 03205 Bronkow (DE); MILIUS, Stephan, 02991 Lauta Dorf (DE); BERGER, Enrico, 01968 Senftenberg Niemtsch (DE)
(74) Vertreter: Gulde & Partner

(57) **Zusammenfassung**

Die Erfindung betrifft ein Verfahren zur Immobilisierung von körpereigenen kernhaltigen Zellen aus Körperflüssigkeiten umfassend einen für spezifische körpereigene kernhaltige Zellen adhäsiven Probenträger, ein Verfahren zur Herstellung eines solchen Probeträgers, sowie ein Kit umfassend einen solchen Probenträger.

## Beschreibung

Die Erfindung betrifft ein Verfahren zur Immobilisierung von körpereigenen kernhaltigen Zellen aus Körperflüssigkeiten umfassend einen für spezifische körpereigene kernhaltige Zellen adhäsiven Probenträger, ein Verfahren zur Herstellung eines solchen Probeträgers, sowie ein Kit umfassend einen solchen Probenträger.

Die Analyse von Zellen, die physiologisch oder pathophysiologisch in Körperflüssigkeiten vorkommen, setzt meist vor der Analyse die Anreicherung oder Reinigung dieser Zellen voraus. Eine der wichtigsten Probenmatrices für Analysen ist Blut, welches häufig z.B. im Rahmen der medizinischen Diagnostik auf zytologische, immunologische oder molekularbiologische Marker untersucht werden muss. Es wurden vielfältige Verfahren entwickelt, um gezielt einzelne Populationen der Blutzellen wie z.B. Erythrozyten oder Leukozyten selektiv zu extrahieren. Die ersten Verfahren die hierfür zum Einsatz kamen waren z.B. Leukozytensedimentationstechniken (Garcia A, Niubo J, Benitez MA, Viquera M, Perez JL. Comparison of two leukozyte extraction methods for zytomegalovirus antigenemia assay. J Clin Microbiol 1996, 182-184) oder Lysetechniken bei denen z.B. die Erythrozyten zerstört werden, um diese von den Leukozyten zu trennen.

Durch Lyse kann auch die Freisetzung der RNA aus den Leukozyten durch Zugabe von Wasser und Diethylpyrocarbonate (Shi YJ, Liu, JZ. Direct reverse trancription-polymerase chain reaction from whole blood without RNA extraction. Genet Anal Tech Appl 1992, 9, 149-150.) erreicht werden. Ein weiteres Beispiel für die Anreicherung oder Abreicherung von Leukozyten aus Blut, ist die Verwendung von Dynabeads, die an der Oberfläche einen Anti-CD45-Antikörper tragen. Ebenso kann die Anreicherung durch mikrofluidische Verfahren mittels Margination in dünnen Kappillaren erfolgen (Shevkoplyas SS, Yoshida T. Bitensky W. Biomimetic autoseparation of Leukocytes from whole blood in microfluidic device. Anal Chem 2005, 77, 933-937; Choi J, Hyun J, Yang S. On-chip extraction of intracellular molecules in white blood cells from whole blood. Scientific Reports 2015, 5, Article Number: 15167) oder auch im Zellsorter (FACS).

Eine Alternative stellt die komplette Auflösung der zu analysierenden Zellen dar, um molekulare Komponenten wie Nukleinsäuren oder Proteine anzureichern und/oder zu reinigen (Thatcher SA. DNA/RNA Preparation for Molecular Detection. Clinical Chemistry 2015. 61:189-199. Tan SC, Yiap BC. DNA, RNA, and Protein Extraction: The Past and The Present. J Biomed Biotechnol, 2009, Article ID 574398).

Die Reinigung soll dazu beitragen, dass störende Komponenten die nachfolgenden Analysen der isolierten Biomoleküle nicht negativ beeinflussen. Eine störende Komponente kann z.B. Immunglobulin G sein, was ein wesentlicher Inhibitor für die Polymerase-Kettenreaktion (PCR) aus Serum ist (Abu Al-Soud W, Jönsson LJ, Radström P. Identification and characterization fo immunglobulin G in blood as a major inhibitor of diagnostic PCR. J Clinical Microbiol 2000, 38, 345-350).

Andere Störfaktoren für molekulare Nachweise können Hämoglobin und Laktoferin aus Erythrozyten bzw. Leukozyten sein. Diese verursachen u.a. bedingt durch Eisen, welches beide Proteine enthalten, aber auch durch Abbauprodukte des Häms, eine Inhibition der PCR (Abu-Al-Soud, Radström P. Purification and characterization of PCR-Inhibitory components in blood. J clin Micorbiol 2001, 39, 485-493). Der Einsatz von Nukleinsäurereinigungskits vor der Durchführung der meisten molekulardiagnostischen Tests entspricht dem heutigen Standard. Die Reagenzienkosten für diese Kits sowie Kosten für die Durchführung der Nukleinsäurereinigung sind jedoch ein beachtlicher Teil der Gesamtkosten jeder molekularen Analyse.

Der manuelle Aufwand konnte durch die Automatisierung der Nukleinsäurereinigung stark vermindert werden, doch wiegen höhere Reagenzienkosten und die Kosten für die Automatisierungstechnik diesen Vorteil teilweise wieder auf. Der direkte Einsatz von Blut in der PCR mittels Blood direct PCR Kit unter Verwendung speziell formulierter Polymerasen und Puffer (Zhang Z, Kermekchiev MB, Barnes WM. Direct DNA amplification from Crude clinical samples using PCR enhancer cocktail and novel mutants of Tac. J Mol Diagn 2010, 12, 152-161.) stellt eine folgerichtige Möglichkeit dar, diese Kosten zu minimieren, hat sich jedoch für diagnostische Anwendungen bislang nicht durchgesetzt. Als Enhancer zur Verbesserung der PCR-Effizienz nutzten Zhang et al. Nonident, Trehalose und Carnitin und außerdem Omni Klentaq o. Omni Taq, was die Flexibilität von Direkt-PCR-Protokollen bei der Wahl der Reaktionsbedingungen während der Testentwicklung deutlich einschränkt und nicht für alle Inhibitoren und Targets zum Erfolg führt. Als nachteilig bei der Direct-PCR mit Blut ist auch die Schwierigkeit zu nennen, die Targetnukleinsäuren für die PCR aus den Blutzellen freizusetzen.

Im einfachsten Falle wurde zur Lyse der isolierten Leukozyten, das Blut eingefroren und aufgetaut, was die Nukleinsäureausbeute für die PCR erhöhte. Zum Teil ist es erforderlich präzipitiertes Blut durch Zentrifugation zu entfernen (Burkhardt J. Amplification of DNA from whole blood. PCR Methods abd Applications 1994, 3, 239-243). Werden die Erythrozyten lysiert, dann ist nach mehreren Waschschritten der verbleibenden Leukozyten ebenfalls die Real-time-PCR im Rahmen molekulargenetischer Analysen möglich (Martiniez-Serra JM, Robles J, Besalduch J. Fluorescence resonance energy transfer-based real-time polymerase chain reaction method without DNA extraction of F5, F2, F12, MTHFR and HFE. J Blood Med 2014, 5, 99-106). Ebenso können getrocknete Buffy-coat-spots direkt in der Real-time-PCR eingesetzt werden (de Gasperis MR, Caione MD, Concato C, Fiscarelli E, di Pietro N, Salotti V, Putignani L, Menichella D, Callea F. Quantitative recovery of proviral HIV-1 DNA from leukocyctes by dried buffy coat spot method for real-time PCR dtermination. J Virol Meth. 2010, 170, 121-127). Die Herstellung der Spots und das Ausstanzen von Spots aus der Matrix bedeuten jedoch für den Anwender insbesondere bei großen Probenserien einen erheblichen manuellen Aufwand.

Neben der Analyse der aus Körperflüssigkeiten wie z.B. Blut isolierten Zellen in der PCR ist es ebenso möglich, isolierte Zellen zytologisch z.B. nach Fluoreszenz-In-situ-Hybridisierung oder immunzytochemisch zu untersuchen. Es können mit diesen Methoden z.B. die Expression von Genen auf mRNA- und Proteinebene, DNA-Doppelstrangbrüche oder Mutationen untersucht werden. Eine diagnostische Anwendung stellen z.B. Lymphozytentransformationstests zur Aktivitätsmessung von Lymphozyten nach Antigenstimulation dar. In allen Fällen verbinden sich die nötigen Präparationsschritte mit einem deutlichen Zeit-, Material- und Arbeitsaufwand, der die Rentabilität der nachfolgenden Analysen beeinträchtigt.

Nachteile des oben aufgeführten Standes der Technik sind zusätzliche Materialkosten für die Probenvorbereitung sowie manuell aufwändige Präparationsschritte wie z.B. die Zentrifugation im Dichtegradienten unter Einsatz von Zentrifugen oder teurer Verbrauchsmittel bei automatisierten Verfahren zur Nukleinsäurereinigung. Die gereinigten Biomoleküle müssen vor der Analyse aus den Gefäßen, in denen die Extraktion erfolgte, in die Reaktionsgefäße überführt werden, was zusätzlich eine Logistik für den Transfer und gegebenenfalls die Lagerung erforderlich macht. Die Zellen müssen lysiert werden, um die Nukleinsäuren für die nachfolgenden molekularen Analysen wie z.B. PCR oder DNA-Sequenzierung überhaupt freisetzen zu können. Das Freisetzen birgt das Risiko, dass die Nukleinsäuren und hier insbesondere RNA während der Probenvorbereitung degradiert werden. Um die Lyse zu ermöglichen und die Degradation zu vermeiden, sind zusätzliche Reagenzien wie Proteasen oder chaotrope Salze erforderlich, die von den zu reinigenden Biomolekülen möglichst rückstandsfrei abgetrennt und im Nachgang entsorgt werden müssen bzw. zusätzliche Reagenzienkosten bewirken.

Der Erfindung liegt nun die Aufgabe zugrunde, die Probleme des Stands der Technik zu vermeiden oder zumindest zu reduzieren. Insbesondere sollen bei der Probenvorbereitung zur Durchführung einer PCR zusätzliche Lyseschritte vermieden werden können, die Durchführung auf wenige einfache Arbeitsschritte begrenzbar sein, keine toxischen Reagenzien für z.B. die Zelllyse oder die Waschschritte verwendet werden müssen und geringe Materialkosten der bereitgestellten Reaktionsgefäße und Verbrauchsmaterialien sicher gestellt sein. Wünschenswert ist zudem ein möglichst einfaches Wegwaschen störender Komponenten aus der Probenmatrix in dem Maße zu ermöglichen, wie es für die jeweilige nachfolgende Analyse erforderlich ist.

Diese Aufgabe wird durch ein Verfahren zur Immobilisierung von körpereigenen kernhaltigen Zellen aus Körperflüssigkeiten, ein Verfahren zur Herstellung eines Probenträger, einen mit dem Verfahren hergestellten Probenträger sowie ein Kit zur Durchführung des erstgenannten Verfahrens mit den Merkmalen der unabhängigen Ansprüche gelöst.

Somit betrifft ein erster Aspekt der Erfindung ein Verfahren zur Immobilisierung von körpereigenen kernhaltigen Zellen (Eucythen). Das Verfahren umfasst erfindungsgemäß in einem i-ten Schritt das Bereitstellen einer Eucythen aufweisenden Körperflüssigkeit, welche in einem ii-ten Schritt der Art mit einem Probenträger in Kontakt gebracht wird, dass eine für spezifische Eucythen adhäsive Oberfläche des Probenträgers mit der Körperflüssigkeit benetzt wird.

Unter adhäsiv ist vorliegend eine Oberfläche zu verstehen, welche ausgebildet ist mit Eucythen, also kernhaltigen Zellen, eine Bindung einzugehen, so dass die Zellen ausgerichtet oder willkürlich auf der Oberfläche haften und insbesondere in Waschungsprozessen nicht oder höchstens teilweise, sowohl bezogen auf die Anzahl, als auch auf Teile von Zellen, von der Oberfläche heruntergespült werden. Die vorgenannte Bindung kann beispielsweise kovalent sein. Alternativ handelt es sich bei der Bindung eher um koordinierende Wechselwirkungen wie beispielsweise van der Waals-Kräfte, Schwefelbrücken oder Liganden Koordinierung. Ferner ist bevorzugt, dass es sich bei den koordinierenden Wechselwirkungen um ausreichend starke elektrostatische oder polare Wechselwirkungen handelt.

In einem iii-ten Schritt wird die Oberfläche von der überstehenden Flüssigkeit getrennt und die Oberfläche des Probenträgers in einem iv-ten Schritt gewaschen, wobei erfindungsgemäß die kernhaltigen Zellen auf der Oberfläche verbleiben. An die die Schritte i bis iv umfassende Probenvorbereitung schließt sich ein Analyseschritt an, der insbesondere auf der Oberfläche des Probenträgers stattfindet.

Ein Vorteil der Erfindung im Vergleich zu handelsüblichen Probenvorbereitungsbestecken ist die Eigenschaft, dass der Probenträger gleichzeitig das Reaktionsgefäß für die nachfolgende Analyse der Zellen oder der Bestandteile dieser Zellen sein kann. Damit ist es nicht nötig, zusätzliche Gefäße für die Probenvorbereitung bereitzustellen noch die vorbereiteten Proben in das Reaktionsgefäß zu überführen. Es ist jedoch ebenso möglich, die immobilisierten Zellen vom Probenträger zu lösen und vor der Analyse in ein anderes Analysegefäß zu übertragen sowie insbesondere bei Teilung der Proben für mehrere Analysen, auf mehrere Reaktionsgefäße zu verteilen. Reaktionsgefäße umfassen Standardformate in der Labordiagnostik. Diese passen zum einen in die meisten Analysegeräte für unterschiedlichste Analysen und können durch Standardliquidhandlingsysteme automatisch bearbeitet werden. Darauf baut unmittelbar ein weiterer Vorteil des erfindungsgemäßen Verfahrens auf, dass durch die Bindung von Zellen aus komplexen Gemischen, wie Körperflüssigkeiten, gezielt Zellen oder zelluläre Bestandteile der Probe ausschließlich durch wenige Pipettierschritte angereichert werden können. Andere Arbeitsschritte wie Spotherstellung und Ausstanzen von Spots, Zentrifugation, Magnetseparation oder Probentransfer zwischen verschiedenen Gefäßen entfallen. Alle Arbeitsschritte, das Kontaktieren des Trägers mit der Probe, das Entfernen der Probe, Waschschritte, gegebenenfalls die Lyse von Zellen sowie das Hinzufügen der Reagenzien für die Analyse sind ausschließlich durch Pipettieren auszuführen und somit sehr leicht automatisierbar. Dies gilt insbesondere für Vollautomaten, die integriert die Probenvorbereitung als auch die nachfolgende Analyse ausführen.

Bevorzugt handelt es sich bei dem Probenträger um Reaktionsgefäße wie Mikrotitrierplatte, Mikroanalyseplatten, Deckgläschen, Objektträger, Glasstäbe, Bechergläser, Kavitäten oder dergleichen mehr. Alternativ kann der Probenträger auch als Plättchen ausgeformt sein, insbesondere zusätzlich ein solches umfassen, welches weiter bevorzugt als Netz oder Sieb ausgeführt ist. Diese Ausgestaltung erhöht die funktionelle Oberfläche des Probenträgers und damit die Ausbeute, wobei gleichzeitig die Dauer des Verfahrensschritte ii) reduziert werden kann. Letzteres ist insbesondere dann der Fall, wenn der netz- oder siebartige Teil des Probenträgers durch die Flüssigkeit hindurch bewegt oder die Flüssigkeit durch den sieb- oder netzartigen Teil hindurch bewegt wird.

In bevorzugter Ausgestaltung der Erfindung ist vorgesehen, dass der Probenträger ein Polyolefin, insbesondere auszuwählen aus der Gruppe Polyethylen, Polypropylen, Polybutylen und/oder deren Copolymere umfasst oder aus einem solchen besteht. Derartige Materialien zeichnen sich vorteilhafter Weise durch eine Oberfläche aus die mit kernhaltigen Zellen koordinative Wechselwirkungen eingeht, so dass ein diese Materialien umfassender Probenträger bereits eine Oberfläche aufweist, die zumindest zu einem geringen Maße für kernhaltigen Zellen adhäsiv wirkt.

Alternativ oder zusätzlich umfasst der Probenträger bevorzugt Polycarbonat, Polysterol, Polymethacrylat, Copolymere aber auch aus Glas, Metall oder Keramik. Vorteilhafter Weise ist das Material des Probenträger zudem optisch transparent, semitransparent oder nicht transparent, weiß oder schwarz oder andersfarbig ausgeführt. Transparentes Material empfiehlt sich insbesondere dann, wenn die Auswertung der nachfolgenden Analyse durch den Träger hindurch, z. B. durch inverse Mikroskopie, erfolgen soll.

Eine Reduzierung einer aggressiven Wirkung bzw. die Erhöhung der adsorptiven Wirkung der Oberfläche auf kernhaltige Zellen wird beispielsweise durch Anwesenheit funktioneller Gruppen der betreffenden Oberfläche erreicht. Derartige Gruppen sind insbesondere Carboxyl-, Hydroxyl-, Amino-, Sulfhydryl-, Epoxy- und Aldehydgruppen. In Abhängigkeit der konkreten Anwendung sind dem Fachmann weitere Möglichkeiten bekannt, die funktionellen Gruppen auf der Oberfläche des Trägers zu gestalten. Ebenso ist es möglich Hydrogele auf der Oberfläche der Träger z.B. durch Polyacrylsäure oder Polyethylenglykol aufzubringen oder durch Einbringen funktionalisierter bzw. funktionalisierbarer Vergussmassen einzubringen.

Die Vergussmassen können Einkomponenten- oder Mehrkomponenten-Vergussmassen sein. Die Aushärtung kann durch Mischen der Komponenten vor dem Einbringen bzw. durch UV-Licht oder Temperatureinwirkung nach dem Einbringen initiiert werden. Das Einbringen von Vergussmassen hat den Vorteil, dass vielfältige Materialien, aus denen die Reaktionsgefäße bestehen können auf diese Weise einfach mit einer universellen Funktionalisierung versehen werden können. Die eingebrachten Vergussmassen können als dünner Film, als Spots oder als teilweise Verfüllung des Reaktionsraumes eingebracht werden. Als Vergussmassen werden bevorzugt Stoffe ohne störende oder mit nur geringen Eigenfluoreszenzeigenschaften wie z.B. transparente Silicone, Epoxidharze, Methacrylate oder Polystyrol verwendet. Diese können selbst funktionalisiert aber auch mit funktionalisierten Komponenten, Stoffen oder Partikeln dotiert sein.

Mit besonderem Vorteil weist die Oberfläche zumindest ein für bestimmte Eucythen spezifisches Fängermolekül, insbesondere ein Histon und/oder ein Phythaemagglutinin-L, auf. Derartige Fängermoleküle sind hierzu auf der Oberfläche des Probenträgers immobilisiert, das heißt derart fixiert, dass sie durch üblichen Gebrauch wie beispielsweise waschen oder benetzten mit Flüssigkeiten, tempern oder ähnlichem nicht von der Oberfläche gelöst werden.

Vorzugsweise sind die Fängermoleküle fest auf der Oberfläche des Trägers immobilisiert, was durch deren kovalente und/oder nicht-kovalente Bindungen erreicht wird. Dadurch wird erreicht, dass auch die später gebunden Zellen während der Waschschritte auf dem Träger immobilisiert bleiben. Bezogen auf Leukozyten wird durch den Einsatz von gezielten Fängermolekülen die Adsorption, also die adhäsiven Eigenschaften auf die spezifischen kernhaltigen Zellen, um ein 10-100-faches gesteigert.

Dem Fachmann sind viele unterschiedliche chemische und physikalische Verfahren bekannt, mit denen Fängermoleküle, die in einem Inkubationsmedium gelöst vorliegen, an die Oberfläche eines Trägers gebunden werden können. Die hier dargestellten Varianten repräsentieren eine Auswahl bevorzuger Verfahren des Standes der Technik:
(a) Adsorption über hydrophobe oder ionische Wechselwirkung aus der Flüssigphase,
(b) Adsorption entsprechend (a) durch Antrocknung,
(c) Kovalente Bindung entsprechend (a) oder (b) durch vorgeschaltete, simultane oder nachgeschaltete UV-Licht-Aktivierung der Oberfläche des Trägers,
(d) Kovalente Bindung durch Aktivierung von Carboxylgruppen auf der Oberfläche des Trägers mittels Kopplungsreagenzien wie 1-Ethyl-3-(3-dimethylaminopropyl)carbodiimid (EDC) oder Succinimid,
(e) Nicht-kovalente Kopplung von biotinylierten Fängermolekülen vermittelt über am Träger entsprechend (a-d) und (f) immobilisiertes Avidin oder Streptavidin,
(f) Kovalente Bindung über aktive Gruppen wie Epoxy- oder Aldehydgruppen auf der Trägeroberfläche bzw. über UV- oder thermisch aktivierbare chemische Gruppen.

Die Fängermoleküle, die nicht am Träger gebunden haben, können je nach Anwendung im Reaktionsgefäß in Lösung verbleiben, entfernt werden oder bevorzugt eingetrocknet werden. Die Stabilisierung mit handelsüblichen Stabilisatoren ist ebenso möglich.

Das Kontaktieren der auf dem Träger immobilisierten Fängermoleküle erfolgt z.B. durch Pipettieren von bevorzugt unverdünnten Proben/Körperflüssigkeiten wie Blut, Serum, Plasma, Tränenflüssigkeit, Urin, Liquor cerebrospinalis, Lymphe, Schweiß, Sputum, Exhalt, Sperma in das Reaktionsgefäß. Ebenso können auch verdünnte Proben wie suspendierter Stuhl oder ausgeschüttelte Abstrichproben in das Reaktionsgefäß pipettiert werden. Bei der Verwendung von Blut als Probe werden bevorzugt Heparin-, EDTA- oder Citrat-Blut zur Verhinderung der Blutgerinnung im Proben- oder Reaktionsgefäß eingesetzt.

Als Fängermoleküle für kernhaltige Zellen werden bevorzugt Proteine, Antikörper, monoklonale Antikörper, rekombinante Antikörper oder Antikörperfragmente, Lektine, Rezeptoren, Komplementproteine, Gerinnungsfaktoren, Histone, Histokompatibiltätsantigene, Nukleinsäuren, künstliche Nukleinsäuren, Aptamere, Peptide, Polyanionen, Polyacrylsäure, Polykationen, Polylysin, Monosaccharide, Oligosaccharide, Polyethylenglykol, oder Lipide oder Kombinationen von zwei oder mehrerer dieser Stoffe, die durch ihre Bindungsspezifität selektiv eine oder mehrere Populationen der Zellen aus der Körperflüssigkeit am Träger immobilisieren bzw. die unspezifische Bindung von Zellen oder Inhibitoren unterdrücken, eingesetzt.

Im spezifischen Fall von Leukozyten als kernhaltige Körperzellen, die insbesondere aus Blut oder anderen Körperflüssigkeiten am Träger gebunden werden, werden als Fängermoleküle bevorzugt Anti-CD45-Antikörper, Histone und Phythaemagglutinin-L einzeln oder gemeinsam auf der luminalen Oberfläche des Trägers oder der Seite des Trägers immobilisiert, die mit der Probe in Kontakt kommt. Alternativ oder zusätzlich werden, Antikörper eingesetzt, die für einzelne Subpopulationen der Leukozyten, z.B. Granulozyten oder T-Helferzellen, spezifisch sind, um diese Subpopulationen anzureichern.

In weiterer bevorzugter Ausgestaltung der Erfindung ist vorgesehen, dass die Körperflüssigkeit eine dem Körper direkt entnehmbare Flüssigkeit, insbesondere Blut, ist. Dies hat den Vorteil, dass die Flüssigkeit leicht zugänglich ist, da sie dem Körper direkt entnommen werden kann. Darüber hinaus wird in dieser Ausgestaltung der Erfindung so zu sagen direkt das Rohmaterial, nämlich die Körperflüssigkeit welche die zu immobilisierenden Zellen aufweist, mit dem Probenträger in Kontakt gebracht, wodurch aufwändige Vorbereitungsschritte entfallen können.

Somit ist bevorzugt, dass die beiden Schritte i) und ii) direkt aufeinanderfolgen und insbesondere zwischen den beiden Schritten kein Lyse-Schritt erfolgt. Überraschenderweise zeigte sich, dass mit der Auswahl einer spezifisch adhäsiven Oberfläche des zu verwendenden Probenträgers eine spezifische Immobilisierung erfolgt. Diese zeigte sich unabhängig von den Begleitstoffen und deren Konzentration. Dies führt mit Vorteil zu einer enormen Kosten- und Zeitersparnis sowie zu einer verbesserten Ausbeute.

Unter Analyseschritt ist vorliegend jede dem Fachmann bekannte Analysemethode für Zellen zu verstehen, wie beispielsweise eine *in situ*-Hybridisierung, eine Polymerasekettenreaktion (PCR), eine *insitu-PCR,* eine Immunzytochemie Zellkultur oder dergleichen, wobei die Durchführung einer Polymerasekettenreaktion als Teil des erfindungsgemäßen Verfahrens bevorzugt ist. Mit besonderem Vorteil erfolgt im Schritt v) eine qualitative Echtzeit Polymerase Kettenreaktion, eine sogenannte *realtime* PCR. Die erfindungsgemäße Immobilisierung von Eucythen aus Körperflüssigkeiten zeigte insbesondere bei diesen Folgeverfahren große Erfolge.

In weiter bevorzugter Ausgestaltung der Erfindung ist vorgesehen dass die Polymerase Kettenreaktion auf dem Probenträger selbst durchgeführt wird. Dies hat den Augen fälligen Vorteil einer Material- und Kostenersparnis sowie einen Gewinn an Ausbeute.

Besonders bevorzugt weist der Probenträger weitere Fängermoleküle auf. Je nach Verfahrensziel sind die weiteren Fängermoleküle auf die gleiche Art Zellen spezifisch oder aber auf eine andere Art. So kann zum einen die Ausbeute gleicher Zellen erhöht werden oder aber, insbesondere wenn die Fängermoleküle abschnittsweise sortiert nebeneinander angeordnet sind, gleichzeitig mehrere Zellarten immobilisiert werden.

Die Ausbeute gleicher Zellen kann weiter erhöht werden, indem der Körperflüssigkeit vor, oder während Schritt ii) funktionalisierte Partikel zugesetzt werden. Dabei wird die Funktionalisierung insbesondere durch Anordnung von Fängermolekülen auf der Partikeloberfläche erreicht. Die Partikel haben bevorzugt Mikro- oder Nanogröße, weisen also bevorzugt einen Partikeldurchmesser im Bereich von 0,5-20 µm (Mikropartikel) oder kleiner (Nanopartikel) auf.

Die Partikel können als Magnetpartikel, fluoreszenzkodierte, größen- oder formkodierte Partikel bereitgestellt werden, die eine Oberflächenfunktionalisierung besitzen, über die zweite Fängermoleküle gebunden sind. Zweite Fängermoleküle können den gleichen Molekülklassen wie erste Fängermoleküle entstammen, zeichnen sich durch eine gleiche aber bevorzugt durch eine andere Bindungsspezifität in Bezug auf Zellen sowie Bestandteile von Zellen oder abgegebenen Stoffen von Zellen aus.

Ferner werden die Partikel bevorzugt vor, während oder nach dem Kontaktieren der Probe mit dem Träger im Reaktionsgefäß zur Probe hinzugegeben. Diese binden in der Folge Zellen, Bestandteile von Zellen oder von Zellen abgegebene Stoffe über zweite Fängermoleküle. Durch magnetische, gravimetrische Sedimentation oder durch Flüssigkeitsströmung im Reaktionsgefäß gelangen dann die Mikropartikel in Kontakt mit dem Träger, wobei sie über die ersten Fängermoleküle direkt oder aber indirekt über die zuvor an die Partikel gebundenen Zellen, Bestandteile oder abgegebenen Stoffe, an den Träger binden. Durch diese Verfahrensausgestaltung ist es möglich, Zellen, Bestandteile oder abgegebenen Stoffe am Probenträger besonders stark anzureichern bzw. für die nachfolgende Analyse zugänglich zu machen. Dieses Verfahren kann z.B. eingesetzt werden, um zirkulierende Tumorzellen im Blut zu analysieren bzw. um fetale Zellen aus dem Blut der Mutter zu analysieren. Durch Verwendung von Partikeln unterschiedlich kodierter Partikelpopulationen ist es möglich, mit jeder Mikropartikelpopulation ein anderes zweites Fängermolekül einzusetzen, um Zellen, Bestandteile sowie abgegebene Stoffe von Zellen in der nachfolgenden Analyse im Multiplexformat wie z.B. bei der Erstellung von Blutbildern oder Multiplex-Antikörpernachweisen zu untersuchen.

Ein weiterer Aspekt der Erfindung ist ein Verfahren zur Herstellung eines Probenträgers aufweisend eine für körpereigene kernhaltige Zellen adhäsive Oberfläche zur Verwendung in dem vorgeschriebenen erfindungsgemäßen Verfahren. Erfindungsgemäß umfasst das Verfahren zunächst ein Benetzen einer Oberfläche eines Precursors eines Probenträger mit einer Lösung, welche zumindest eine Sorte spezifischer Fängermoleküle für einen korrespondierenden Zelltyp umfasst. Dabei wird unter Precursor eine Vorstufe oder ein Vorläufer für einen erfindungsgemäßen Probenträger verstanden, dessen Oberfläche noch nicht die erfindungsgemäßen spezifischen adhäsiven Eigenschaften aufweist. Er kann aber bereits intrinsich für kernhaltige Zellen adhäsive aber auch abstoßende Eigenschaften haben. Diese Vorstufe wird nach dem Benetzen mit ultravioletten Licht, also mit Licht in einem Wellenlängenbereich von 100-400 nm, insbesondere in einem Bereich von 200-400 nm vorzugsweise im Bereich von 280-380 nm, bestrahlt. Dabei kommt es zu einem Vernetzen der Fängermoleküle mit der Oberfläche des Probenträgers. Die vernetzte Oberfläche wird abschließend getrocknet.

Ein derart hergestellter Probenträger zeigt die erfindungsgemäßen spezifischen adhäsiven Eigenschaften auf einer Gruppe kernhaltiger körpereigener Zellen und kann somit im vorgenannten erfindungsgemäßen Verfahren zur Immobilisierung derartiger Zellen verwendet werden.

Mit Vorteil weist die Lösung ein Histon und/oder einPhythaemagglutinin auf. Diese zeigten sich insbesondere bei der Verwendung von Blut als Körperflüssigkeit zur selektiven Immobilisierung von Leukozyten als besonders vorteilhaft. Wobei sich vorteilhaft auf die Spezifität und die Ausbeute der immobilisierten Leukozyten bezieht.

Weiter vorteilhaft ist, wenn der Vorläufer des Probenträgers zu Beginn des Verfahrens einen Haftvermittler für Fängermoleküle aufweist. Geeignete Haftvermittler sind dem Fachmann bekannt, besonders bevorzugt zeigte sich die Verwendung von carboxymethyliertem Dextran oder Polyacrylsäure. Die Verwendung eines Haftvermittlers erhöht die Bindung zwischen Fängermolekül und Probenträgeroberfläche.

Ein weiterer Aspekt der Erfindung betrifft einen Probenträger aufweisend eine für kernhaltige körpereigene Zellen adhäsive Oberfläche hergestellt oder herstellbar mit dem vorgenannten erfindungsgemäßen Verfahren. Weiter betrifft die Erfindung ein Kit zur Durchführung eines erfindungsgemäßen Verfahrens zur Immobilisierung von körpereigenen kernhaltigen Zellen aus Körperflüssigkeiten, welches einen erfindungsgemäßen Probenträger umfasst.

Mit Vorteil umfasst das erfindungsgemäße Kit zusätzlich geeignete Waschlösungen, die einen benutzten Probenträger insbesondere im Verfahrensschritt iv) von überstehender Flüssigkeit und darin gelöst oder sedimentierten Zellen oder Partikeln befreit, aber dennoch keine Bindung mit den immobilisierten Zellen bzw. Fängermolekülen eingeht und deren Haftung auf der Probenträgeroberfläche nicht störend beeinflusst, wobei störend vorliegend dahingehend zu verstehen ist, dass dadurch die nachfolgende Analyse der Zellen oder ihrer Bestandteile relevant im Hinblick auf Sensitivität und Spezifität verschlechtert wird.

Weitere bevorzugte Ausgestaltungen der Erfindung ergeben sich aus den übrigen, in den Unteransprüchen genannten Merkmalen.

Bei den erfindungsgemäßen Verfahren werden insbesondere die Verwendung der Materialien in den oben beschriebenen Ausführungen bevorzugt. Mit anderen Worten alle voranstehenden Gegenstände und Verwendungen der Erfindung und deren Ausführungsformen sind vorteilhaft zu kombinieren. Zudem betreffen die Ausführung zu bestimmten Gegenständen und Verwendungen der Erfindung stets auch die anderen Gegenstände und Verwendung und deren Ausführungsformen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine fluoreszenzmikroskopische Aufnahme von immobilisierten Leukozyten nach einer bevorzugten Ausgestaltung des erfindungsgemäßen Verfahrens,
- Figur 2: eine grafische Gegenüberstellung in Form von Fluoreszenzzyklen in Abhängigkeit einer Lagerung einer erfindungsgemäßen Probe mit einer Probe zum Stand der Technik.

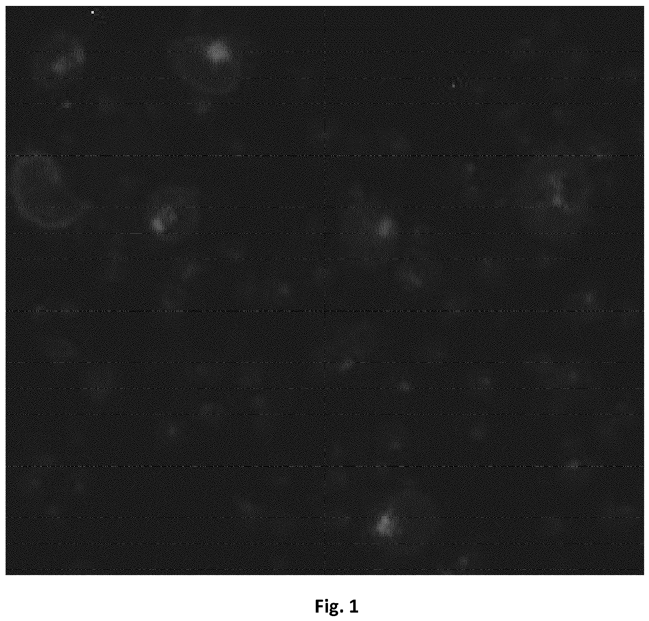

Figur 1 zeigt eine fluoreszenzmikroskopische Aufnahme von immobilisierten Leukozyten aus humanem Blut welche durch die Fängermoleküle Histon und Phythaemagglutinin-L an einen erfindungsgemäßen Träger einer Flachbodenkavität (Mikrotestplatte) gebunden und durch Phythaemagglutinin-L-Atto647N markiert wurden. Die Auswertung erfolgte mittels Fluoreszenzmikroskop IX81 (Olympus).

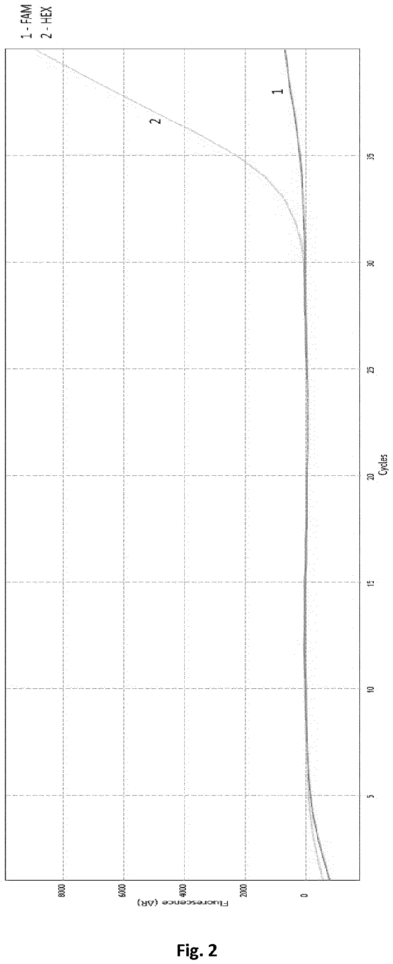

Figur 2 zeigt Zellen welche entsprechend Figur 1 in einem PCR-Tube immobilisiert und gewaschen wurden. Danach erfolgte ein Mutationsnachweis einer homozygot mutierten Probe unter Verwendung eines kommerziellen Kits (attomol-MTHFR 1298A>C Realtime TM, #1202) in Kombination mit dem Real-time-PCR-Cycler AriaMX (Agilent). 1-FAM: Signalverlauf der Wildtyp-spezifischen Sonde; 2-HEX: Signalverlauf der mutationsspezifischen Sonde.

Es zeigte sich, dass nach einer Inkubation von 0,5-20 Minuten im Falle von Blut als Probenmaterial bereits so viele Leukozyten aus dem Blut an den Träger gebunden waren, dass fast eine weitgehend geschlossene Zellayer vorliegt (Fig. 1). Durch Schütteln während der Inkubation kann die Zelldichte weiter gesteigert werden, was jedoch für die meisten Analysen dieser Zellen nicht erforderlich ist. Sind nur sehr wenige Zellen in der Körperflüssigkeit enthalten, wie z.B. bei der Isolation von zirkulierenden Tumorzellen aus Blut, wird im Abstand von bevorzugt 30 sec Intervallschütteln genutzt, um mehr Tumorzellen mit den Fängermolekülen Anti-EPCAM-Antikörper (EPCAM, Epethelial Cell Adhesion Molecule), die zuvor auf dem Träger immobilisiert wurden in Kontakt zu bringen. Die Länge der Intervalle und die Intensität des Schüttelns richten sich nach der jeweiligen Anwendung.

Nach dem Kontaktieren der Fängermoleküle mit der Probe, was in Bezug auf die Art der eingesetzten Probe oder später erfolgenden Analyse unterschiedlich lange dauert, wird die Probe bevorzugt durch einen Pipettierschritt entfernt. Sollte es für die nachfolgende Analyse nötig sein, eine weitere Reduktion der Probenbestandteile vorzunehmen, werden optional Waschschritte mittels einer oder verschiedener Waschlösungen vorgenommen. Ein Schütteln ist meist nicht erforderlich, da durch das Pipettieren der Waschlösung allein bereits eine gute Durchmischung des Mediums erreicht werden kann. Die Waschlösung ist bevorzugt eine pHgepufferte isotonische Waschlösung, wodurch die Zellen vor unnötiger Schädigung geschützt sind. Sollen die Zellen im Rahmen der Analyse z.B. *in vitro* kultiviert oder stimuliert werden, ist ein schonendes Waschen unerlässlich. Andererseits kann es auch das Ziel sein die Zellen vor, während oder nach dem Waschen gezielt zu lysieren. Dies erfolgt bevorzugt durch Zugabe einer hypotonen Lösung wie destilliertem Wasser bzw. alkalischer, saurer,alkoholischer oder hypertoner Lösungen sowie anderer Detergenzien erfolgen. Zwischen dem letzten Waschschritt und der Zugabe des Reaktionsgemisches für die Nachweisreaktion ist es von Vorteil, Flüssigkeitsreste der Waschlösung einzutrocknen bzw. aus dem Reaktionsgefäß heraus zu zentrifugieren. Dies würde die Lyse verstärken und störende Verfälschungen des Reaktionsvolumens, was letztlich zur Verdünnung oder Inhibition des Reaktionsgemisches führen kann, verhindern. Besonders geeignet ist ein thermischer Lyseschritt besonders bevorzugt nach Zugabe der Reaktionslösung für die nachfolgende Analyse z.B. mittels Real-time-PCR. Ein besonderer Vorteil der thermischen Lyse ist, dass keine Lysereagenzien in die Reaktionslösung verschleppt werden. Außerdem bleiben die Zellen und somit die natürlichen Verhältnisse in denen die Analyten vorkommen bis unmittelbar vor der Reaktion erhalten, was deren Kontakt mit degradierenden Enzymen reduziert. Es ist aber genauso auch möglich, Bruchstücke von Zellen oder tote Zellen zu immobilisieren, die länger gelagert oder z.B. zuvor eingefroren wurden. So zeigte sich das Verfahren erstaunlich robust bezüglich der Probenlagerung im Falle von Blut, in dem nach 3 Wochen Lagerung bei 4 °C immer noch korrekte Analysen von humanen Genpolymorphismen durchgeführt werden konnten (Fig. 2). Besonders bevorzugt ist es durch einen oder mehrere Waschschritt(e) in hypotoner Waschlösung die am Träger gebundenen Zellen zu lysieren, um Inhibitoren der Nachweisreaktion aus den Zellen zu entfernen. Hochmolekulare, nichtlösliche oder anderweitig im Zellinneren gebundene Analyten verbleiben bevorzugt in den lysierten Zellen, um die Analyten dann in der Nachweisreaktion zu detektieren. Dabei ist es durchaus vorteilhaft, die Analyten nach dem Waschschritten für die Analyse aus den Zellen herauszulösen.

Sollen die Zellen einer mikroskopischen Analyse zugeführt werden, umfasst das Verfahren die Verwendung von Reaktionsgefäßen, mit planarem, transparentem Boden wie Objektträger oder Mikrotestplatten. Um die Zellen für diesen Anwendungsfall stabilisieren zu können, werden sie bevorzugt durch Verwendung paraformaldehyd-, glutaraldehydhaltiger, oder alkoholischer Lösungen oder durch Antrocknen direkt im Reaktionsgefäß nach deren Immobilisierung auf dem Träger fixiert.

### Ausführungsbeispiele:

### Beispiel 1: Lektinzytochemischer Fluoreszenznachweis

### Präparation des Probenträgers:

- Pipettieren von Histon- und Phythaemagglutinin-L-Lösung in Phosphatpuffer in eine Flachbodenmikrotestplatte aus Polystyrol (Greiner)
- Bestrahlung mit UV-Licht mit einem Wellenlängenbereich von 200-400 nm
- Entfernen der Lösung aus den Kavitäten der Mikrotestplatte
- Trocknung der Mikrotestplatte bei Raumtemperatur

### Immobilisierung von Leukozyten aus Blut:

- Pipettieren von Blut in die Tubes und Inkubation
- Entfernung des Blutes durch einen Pipettierschritt
- dreimaliges Waschen mit 0,1 M Natrium-Phosphatpuffer der 0,9 % NaCl enthält
- Entfernen der Waschlösung
- Zugabe und Inkubation eines Konjugates aus Phythameagglutinin-L und Atto647N
- dreimaliges Waschen mit 0,1 M Natrium-Phosphatpuffer der 0,9 % NaCl enthält
- Auswertung der Zellen im Fluoreszenzmikroskop (siehe Fig. 1).

### Beispiel 2

### Präparation des Probenträgers:

- Pipettieren von Histon- und Phythaemagglutinin-L-Lösung in Phosphatpuffer in PCR-Tubes aus Polypropylen,
- Bestrahlung mit UV-Licht mit einem Wellenlängenbereich von 200-400 nm,
- Enfernen der Lösung aus den PCR-Tubes,
- Trocknung der PCR-Tubes bei Raumtemperatur,

### Immobilisierung von Leukozyten aus Blut

- Pipettieren von Blut in die Tubes und Inkubation,
- Entfernung des Blutes durch einen Pipettierschritt,
- dreimaliges Waschen mit 0,1 M Natrium-Phosphatpuffer der 0,9 % NaCl enthält,
- Entfernen der Waschlösung,
- Zugabe des PCR-Mix entsprechend attomol-MTHFR 1298A>C Realtime TM, #1202,
- Verschließen der Tubes und Überführung in den Real-time-PCR-Cycler und Start der Real-time-PCR(siehe Fig. 2).

Ein besonderer Vorteil des Verfahrens besteht darin, unmittelbar an den isolierten Zellen vielfältige Analysen vornehmen zu können. Dies können mikroskopische Analysen zur Bestimmung der Zytologie der Zellen sein. Es ist dabei möglich gezielt die Expression bestimmter z.B. mRNA- oder Proteinmarker von einzelnen oder allen Zellen mittels in situ-Hybridisierung oder Immunzytochemie zu untersuchen. Es ist möglich DNA-Doppelsträngbrüche in den Leukozyten zu bestimmen, um z.B. die Strahlenbelastung des Patienten einschätzen zu können. Es ist ebenso möglich, lebende Zellen intakt am Träger zu binden und diese im Rahmen einer in vitro-Kultur oder nach einer gezielten Stimulation zu analysieren. Sehr praxisrelevante Beispiele sind Lymphozytentransformationstest oder die Bestimmung der Therapeutikaresistenz von Tumorzellen und Bakterien, die aus dem jeweiligen Probenmaterial wie z.B. Blut, Sputum oder Tumorgewebe an den Träger gebunden werden. Natürlich ist es ebenso möglich, die Zellen vor, während oder nach der Analyse aufzulösen, um bestimmte Analyten für die Analyse oder weitere Analysen zugänglich zu machen. Bei solchen Analysen wie PCR, Real-time-PCR, digitale PCR werden die zu analysierenden Nukleinsäuren bevorzugt durch thermische Lyse der immobilisierten Zellen unmittelbar zu Beginn der Reaktion für die Reaktion zugänglich gemacht und gleichzeitig degradierende Enzyme wie Proteasen und Nukleasen inaktiviert. Das geschieht z.B. bevorzugt während des initialen 95°C-Schrittes, bei dem Hot-Start-Polymerasen bevorzugt gleichzeitig aktiviert werden. Bei der in situ-PCR hingegen, werden die Zellen durch eine chemische Fixierung stabilisiert, so dass die Analyten in den Zellen nachgewiesen werden können und die Information zur Lokalisation der Analyten erhalten bleibt. Neben diesen vielfältigen Anwendungsmöglichkeiten sind dem Fachmann natürlich weitere Ausführungsformen gemäß dem Stand der Technik bekannt. Die Erfindung umfasst die Anwendung des Verfahrens und des Kits für die medizinische und veterinärmedizinische Diagnostik und für die forensische sowie für die Life-Science-Bioanalytik.

Im Vergleich zum Stand der Technik zeichnet sich die offenbarte Erfindung demnach durch folgende Eigenschaften aus:
- Es handelt sich um ein technisch sehr einfaches Probenvorbereitungssystem, was mit nur einem Arbeitsvorgang, dem Pipettieren, Analyten, d.h. Zellen, Bestandteile von Zellen oder von Zellen abgegebenen Stoffen aus der Probe in die Analyse überführt.
- Probenvorbereitung und Analyse finden bevorzugt im selben Reaktionsgefäß statt, was Materialkosten und Arbeitsschritte einspart.
- Die Lyse oder Stabilisierung von Zellen zur Freisetzung oder Freilegung der Analyten, kann variabel in einer auf die jeweilige Analyse abgestimmten Weise erfolgen.
- Zytologische Analysen können mit PCR-Analysen direkt oder nacheinander kombiniert werden.
- Durch Einsatz verschiedener Fängermoleküle und von Mikropartikeln können die Analyten selektiv im Multiplexformat der Analyse zugeführt werden.
- Die thermische Lyse von Zellen während der Analyse, hilft das Ergebnis verfälschende Degradationsprozesse der Probe zu vermeiden.
- Das offenbarte Verfahren ermöglicht die Durchführung und Kombination unterschiedlichster Analyseverfahren für ein und denselben bzw. unterschiedliche Analyten der Probe.

## Patentansprüche

1. Verfahren zur Immobilisierung von körpereigenen kernhaltigen Zellen aus Körperflüssigkeiten umfassend die folgenden Schritte in der angegebenen Reihenfolge:
i) Bereitstellen einer, kernhaltige Zellen (Eucythen) aufweisenden Körperflüssigkeit,
ii) Benetzen einer, für spezifische Eucythen adhäsiven Oberfläche eines Probenträgers mit Körperflüssigkeit,
iii) Trennen der Oberfläche von überstehender Flüssigkeit,
iv) Waschen des Probenträgers,
v) Durchführen eines Analyseschrittes auf der Oberfläche.

2. Verfahren nach Anspruch 1, **dadurch gekennzeichnet, dass** der Probenträger ein Polyolefin, insbesondere auszuwählen aus der Gruppe Polyethylen, Polypropylen, Polybutylen und/oder deren Copolymeren, umfasst oder aus einem solchen besteht.

3. Verfahren nach einem der vorhergehenden Ansprüche, **dadurch gekennzeichnet, dass** die Oberfläche zumindest ein für spezifische Eucythen spezifisches Fängermolekül, insbesondere ein Histon und/oder ein Phythaemagglutinin-L, aufweist.

4. Verfahren nach einem der vorhergehenden Ansprüche, **dadurch gekennzeichnet, dass** in Schritt i) oder ii) der Körperflüssigkeit Partikel zugegeben werden, die ebenfalls spezifische adhäsive Eigenschaften, insbesondere auf Eucythen, aufweisen.

5. Verfahren nach einem der vorhergehenden Ansprüche, **dadurch gekennzeichnet, dass** die Körperflüssigkeit eine dem Körper direkt entnehmbare Flüssigkeit ist, insbesondere Blut.

6. Verfahren nach einem der vorhergehenden Ansprüche, **dadurch gekennzeichnet, dass** die Schritte i) und ii) direkt aufeinanderfolgen, insbesondere zwischen den Schritten i) und ii) kein Lyse-Schritt erfolgt.

7. Verfahren nach einem der vorhergehenden Ansprüche, **dadurch gekennzeichnet, dass** der Analyseschritt in Schritt v) eine Polymerase Kettenreaktion (PCR) umfasst oder ist.

8. Verfahren nach Anspruch 7, **dadurch gekennzeichnet, dass** der Schritt v) eine quantitative Echtzeit Polymerase Kettenreaktion umfasst.

9. Verfahren nach einem der vorhergehenden Ansprüche, **dadurch gekennzeichnet, dass** der Schritt v) auf demselben Probenträger durchgeführt wird wie die vorhergehenden Schritte ii) bis iv).

10. Verfahren zur Herstellung eines Probenträgers aufweisend eine für spezifische körpereigene kernhaltige Zellen (Eucythen) adhäsiven Oberfläche zur Verwendung in einem Verfahren nach einem der vorhergehenden Ansprüche, das Verfahren umfassend die folgenden Schritte in der angegebenen Reihenfolge:
a) Benetzen der Oberfläche eines Precursors eines Probenträgers mit einer Lösung umfassend zumindest eine Sorte spezifischer Fängermoleküle für einen korrespondierenden Zelltyp,
b) Bestrahlen der benetzten Oberfläche mit ultraviolettem Licht, und
c) Trocknen der Oberfläche.

11. Verfahren nach Anspruch 10, **dadurch gekennzeichnet, dass** die Lösung ein Histon und/oder ein Phythaemagglutinin-L umfasst.

12. Verfahren nach einem der Ansprüche 10 und 11, **dadurch gekennzeichnet, dass** die Oberfläche des Precursors einen Haftvermittler für Fängermoleküle, insbesondere ein carboxylmethyliertes Dextran aufweist.

13. Verfahren nach einem der Ansprüche 10 bis 12, **dadurch gekennzeichnet, dass** der Precursor ein Poleolefin, insbesondere Polyethylen, Polepropylen, Polybutylen, deren Copolymere oder eine Mischung derer aufweist oder daraus besteht.

14. Probenträger aufweisend eine für Eucythen adhäsive Oberfläche hergestellt oder herstellbar nach einem Verfahren gemäß der Ansprüche 10 bis 13.

15. Kit zur Durchführung eines Verfahrens nach einem der Ansprüche 1 bis 9 aufweisend einen Probenträger nach Anspruch 14.
